# EUROPÄISCHE PATENTANMELDUNG

(11) **EP 2 716 327 A1**
(43) Veröffentlichungstag der Anmeldung: **09.04.2014**
(21) Anmeldenummer: 13187238.4
(22) Anmeldetag: 03.10.2013
(51) Int. Cl.: A61N 5/06, H05B 3/00, F21V 17/10

(54) **Infrarotstrahler**

(30) Priorität: 03.10.2012 AT 10672012
(71) Anmelder: B-Intense GmbH, 2201 Gerasdorf bei Wien (AT)
(72) Erfinder: Zimmermann, Andreas, 6166 Fulpmes (AT); Lenhart, Karl, 6511 Zams (AT)
(74) Vertreter: Ofner, Clemens

(57) **Zusammenfassung**

Die Erfindung betrifft einen Infrarotstrahler umfassend mindestens ein Filterglas, mindestens eine Infrarotquelle und mindestens eine Filterglas-Haltevorrichtung. Die Filterglas-Haltevorrichtung dient dazu, mindestens ein Filterglas in einer stabilen Position relativ zur Infrarotquelle zu halten. Sie kann durch Trägerelemente und/oder Teile eines Gehäuses und/oder eines Reflektors des Infrarotstrahlers gebildet sein. Das Filterglas dient zur spektralen Filterung, insbesondere des sichtbaren und/oder des IR-A Anteils, des vom Infrarotemitter emittierten Spektrums. Wesentlich ist dabei, dass mindestens eine Filterglas-Haltevorrichtung mit mindestens einem Filterglas verklebt ist.

## Beschreibung

Diese Erfindung befasst sich mit einem Infrarotstrahler und einem Herstellungsverfahren für einen Infrarotstrahler.

Infrarotstrahler sind Strahler, die mittels einer Infrarotquelle elektromagnetische Strahlung im infraroten (IR) und teilweise auch im sichtbaren Spektralbereich emittieren. Als Infrarotquellen sind verschiedene Systeme wie Flächenstrahler, Keramik- oder Vollspektrumstrahler bekannt.

Infrarotstrahler finden insbesondere Anwendung in Infrarotkabinen, die in den letzten Jahren immer beliebter zur Gesundheitsvorsorge oder einfach nur zur Entspannung geworden sind. Im Gegensatz zu einer Sauna wird in einer solchen Kabine keine Reiztherapie mit starker Belastung des Herz-Kreislauf-Systems, sondern eine milde Anwendung von Wärme angewendet. Typischerweise beträgt die Temperatur in einer Infrarotkabine nur ca. 30 bis 35°C bei geringer Luftfeuchte. Die Benutzung einer Infrarotkabine erfolgt in der Regel in einer entspannten, sitzenden Position vor einem Rückenstrahler. Zur Ergänzung wird der Körper meist auch von vorne über einen weiteren Infrarotstrahler mit Wärme versorgt.

Je nach verwendeter Infrarotquelle (Emitter) unterscheiden sich die Strahler in ihrem Aufbau. Zum Beispiel kann ein Infrarotstrahler ein offenes Gehäuse mit Abschluss durch ein Schutzgitter gegen Berührung der heißen Emitteroberflächen aufweisen.

Beim Einsatz eines Glühdrahtemitters (Vollspektrumstrahler) wird das Gehäuse mit einem Schutzglas (Filterglas) abgeschlossen, das einerseits die Berührung des Emitters verhindert und andererseits den Anteil der Strahlung soweit reduziert, dass keine Blendung oder sonstige strahlungsbedingte Gefahr für das Auge besteht.

Die für solche Strahler verwendeten Filtergläser sind typischerweise aufgrund der geforderten hohen mechanischen und Temperaturschockfestigkeit aus Glaskeramik gefertigt (z.B. Schott Robax® oder Resistan). Die Filterwirkung wird durch eine Färbung des Glases erzielt und kann in gewissen Grenzen variiert werden. Das optische Erscheinungsbild ergibt sich dabei hauptsächlich aus der Lage der kurzwelligen Absorptionsgrenze, mehr oder weniger im sichtbaren Spektralbereich gelegen. Die obere langwellige Grenze des Transmissionsfensters ist hingegen hauptsächlich vor der Glaskeramik an sich und nicht von der Einfärbung gegeben und liegt typischerweise im Bereich um λ = 4,5 µm.

Infrarotstrahler mit einer Abstrahltemperatur unter 1000°K, wie sie zum Beispiel bei Keramikstrahlem gegeben ist, emittieren hauptsächlich im IR-C Bereich (λ = 3 - 1000 µm) und weisen somit keinen oder nur einen unwesentlichen Spektralanteil im IR-A (λ = 0,78 - 1,4 µm) und IR-B (λ = 1,4 - 3 µm) auf. Da die Filtergläser aber im IR-C Bereich so gut wie undurchsichtig sind, können sie nicht in Verbindung mit Temperaturstrahlern mit einer Abstrahltemperatur unter 1000°K verwendet werden.

Sind Infrarotemitter nur mit einem Abdeckgitter geschützt, so können ungehindert Staub, Schmutz oder Schweiß ins Gehäuse des Infrarotstrahlers eindringen und ergeben eine aus hygienischen Aspekten untragbare Situation.

Bei Vollspektrumstrahlern mit wesentlichen IR-A und IR-B Anteilen kann ein Filterglas verwendet werden, welches das Gehäuse gegen das Eindringen von Schmutz schützt. Das Filterglas muss dazu aber mit dem Strahlergehäuse verbunden werden. Dies erfolgt nach dem Stand der Technik durch Einschieben des Glases in eine dafür im Strahlergehäuse vorgesehene Nut oder durch Verklemmen des Glases von Innen gegen eine Nase des Gehäuses.

Nachteil der bisher verwendeten Systeme ist, dass die Anschlussbereiche zwischen Glas und Gehäuse zur einfachen und effektiven Reinigung kaum oder nicht ausreichend zugänglich sind. Nachteilig ist des Weiteren, dass die Befestigung des Glases mit dem Gehäuse einer aufwändigen Konstruktion bedarf, welche zudem die Abmessungen des gesamten Gehäuses vergrößert.

Aufgabe der Erfindung ist es, die Nachteile des Standes der Technik zu überwinden und einen Infrarotstrahler mit einer verbesserten Konstruktion des Strahlergehäuses zur Verfügung zu stellen.

Diese Aufgabe wird durch einen Infrarotstrahler nach den Ansprüchen gelöst, die eine verbesserte Art der Verbindung des Filterglases mit dem Strahlergehäuse aufweisen.

Der erfindungsgemäße Infrarotstrahler umfasst mindestens ein Filterglas, mindestens eine Infrarotquelle und mindestens eine Filterglas-Haltevorrichtung.

Die Filterglas-Haltevorrichtung dient dazu, mindestens ein Filterglas in einer stabilen Position relativ zur Infrarotquelle zu halten. Bevorzugte Filterglas-Haltevorrichtungen sind Trägerelemente und/oder Teile eines Gehäuses und/oder eines Reflektors des Infrarotstrahlers und bestehen bevorzugt aus Stoffen der Gruppe Metall, Holz, Pappe, Glas und Kunststoff.

Die Filterglas-Haltevorrichtung ist direkt mit der Infrarotquelle verbunden oder indirekt über ein Haltesystem, das vorzugsweise Elemente der Gruppe Träger, Gehäuseteile und Reflektoren aufweist.

Eine direkte Verbindung hat den Vorteil, dass je nach Einbau des Infrarotstrahlers keine zusätzlichen Gehäuseteile verwendet werden müssen, was sich positiv auf das Gewicht und den Preis auswirkt. Eine indirekte Verbindung hat den Vorteil eines einfachen, modularen Aufbaus. Zum Beispiel kann ein Gehäuserahmen oder Teile desselben als Filterglas-Haltevorrichtung dienen und die Infrarotquelle an anderen Teilen des Gehäuserahmens (z.B. an der Rückfläche) befestigt sein, wobei diese anderen Teile das Haltesystem bilden. Das Haltesystem und die Filterglas-Haltevorrichtung kann nun auf einfache Weise miteinander verbunden werden, z.B. geschraubt oder mit Federelementen zusammengesteckt, was eine einfache Wartung des Strahlers ermöglichen würde.

Erfindungsgemäß wird die Befestigung eines Filterglases an einer Filterglas-Haltevorrichtung nicht mehr durch Verklemmen des Filterglases erreicht, sondern durch Verklebung mindestens einer Filterglas-Haltevorrichtung mit mindestens einem Filterglas, insbesondere an der Rückseite, der Vorderseite oder den Seitenflächen des Filterglases, wobei in letzterem Falle die Filterglas-Haltevorrichtung vorzugsweise bündig mit der Oberfläche des Filterglases abschließt und insbesondere keine Fuge zwischen Filterglas und Filterglas-Haltevorrichtung vorliegt.

Da die für die Filtergläser hauptsächlich zum Einsatz kommende Glaskeramik selbst eine sehr hohe mechanische Festigkeit aufweist, übernimmt das Glas in einer bevorzugten Ausführungsform auch einfache tragende mechanische Funktionen, zum Beispiel, dass es fest in der Wand einer Infrarotkabine befestigt werden kann und über die Filterglas-Haltevorrichtung selbsttätig die Infrarotquelle und deren Stromanschlüsse stabil hält.

Die Glaskeramik der Filtergläser weist vorzugsweise einen geringen thermischen Ausdehnungskoeffizienten auf. Der typische mittlere lineare thermische Ausdehnungskoeffizient (nach DIN ISO 7991) liegt dabei im Bereich von (-0,4 - 0,9) x 10⁻⁶ K⁻¹ im Temperaturbereich von 20 - 300°C.

Die Verklebung erfolgt mit speziellen temperaturfesten Klebstoffen, welche im abgebundenen Zustand noch einen gewissen Grad an Verformbarkeit aufweisen, um mögliche auftretende temperaturbedingte Ausdehnungen der verwendeten Materialien abfangen und/oder kompensieren zu können. Der zur Verklebung verwendete Kleber bzw. das entsprechende Klebemittel weist im abgebundenen Zustand vorzugsweise noch eine Flexibilität auf.

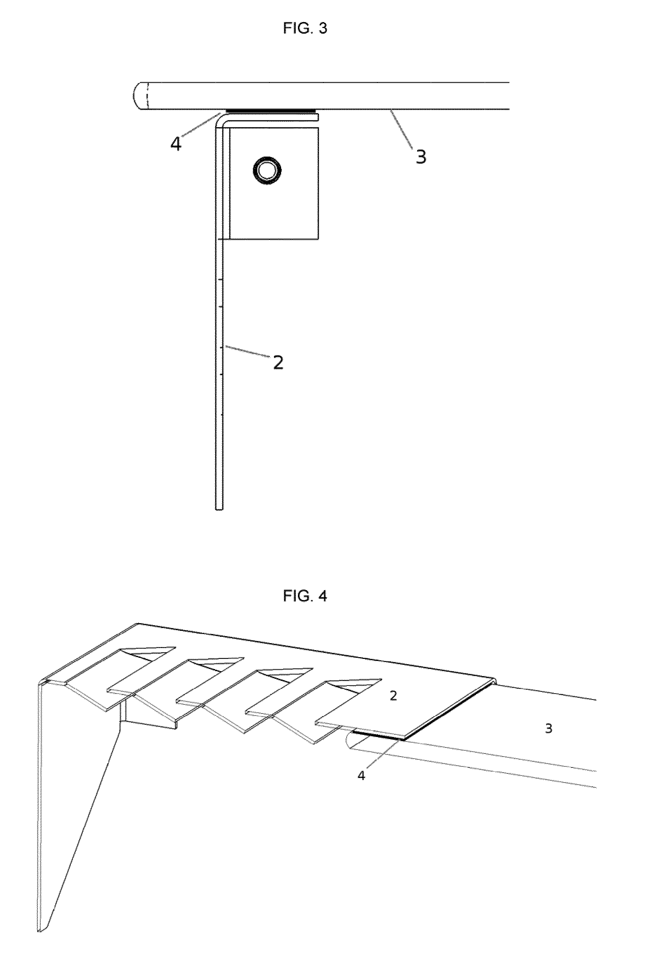

Diese Verklebung ist dabei allgemein zu verstehen. Insbesondere können 2-komponentige Silikonkleber und/oder spezielle Klebebänder zum Einsatz kommen. Entsprechend einer zweckmäßigen Ausführungsform erfolgt die Verklebung der Rückenstrahler gemäß Fig. 3 mit einem Silikonkleber und die Verklebung der Frontstrahler gemäß Fig. 4 mit einem Klebeband mit einer Klebebanddicke von etwa 0,25 mm.

Die Flexibilität bzw. Elastizität der Klebemittel ist dabei derart gewählt, dass beispielsweise der verwendete Silikonkleber eine Bruchdehnung von ca. 150% aufweist. Als Klebebänder eignen sich beispielsweise Hochleistungs-Klebstoff-Filme VHB® der Firma 3M®. Bei unterschiedlichen Längenausdehnungen können diese Klebebänder bis zu 300% ihrer Dicke ausgleichen.

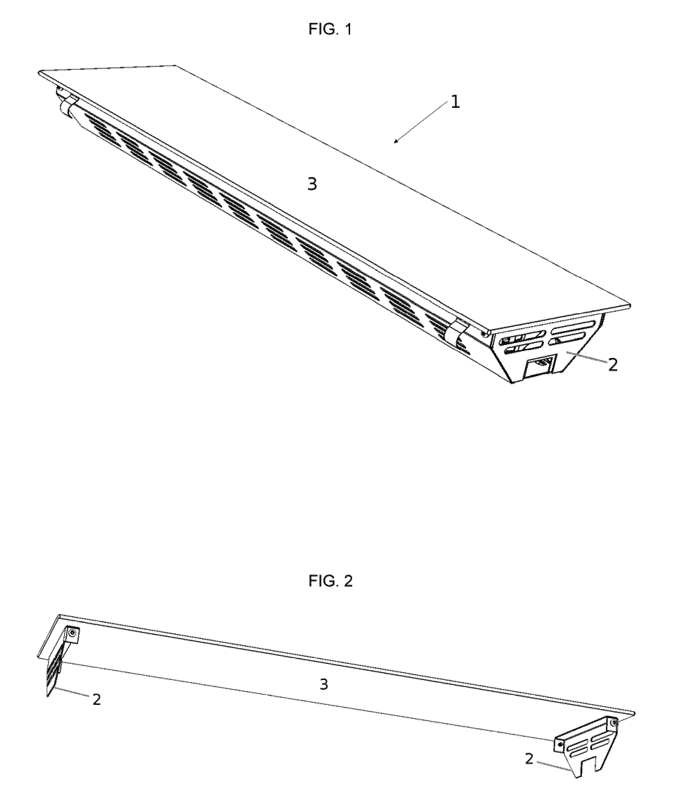

Bei einer bevorzugten Ausführungsform wird die Filterglas-Haltevorrichtung an der Rückseite des Filterglases angeklebt. Mit einer solchen Verklebung ist es erstmals möglich, einen rahmenlosen Infrarotstrahler ohne störenden Rand zu schaffen, welcher sich von Außen nur mehr als eine vollkommen, sich beinahe in die Kabinenwand nahtlos einbindende, glatte Glasfläche darstellt (siehe auch Figur 1).

Bei den bisherigen Gehäusekonstruktionen war es notwendig, das Filterglas an den Längsseiten zu fassen, was ein gewisses Übermaß des Gehäuses mit einem mehr oder weniger störenden Rand (Rahmen) bedingt. Störend sowohl aus designtechnischen Aspekten, aber vor allem auch aus hygienischen Gründen.

Bei einer bevorzugten Ausführungsform wird das Filterglas an seiner Rückseite mit Gehäuseteilen des Infrarotstrahlers verklebt, welche hier die Funktion der Filterglas-Haltevorrichtung übernehmen (siehe auch Figuren 2 und 3). Auf diese Weise wird ein kompakter, rahmenloser Aufbau ermöglicht. Diese Gehäuseteile können dann mit der im Strahler innen liegenden Technik wie Infrarotquelle und der Verkabelung und den restlichen Gehäuseteilen verbunden werden, zum Beispiel durch Verschraubung, und so das Strahlergehäuse abschließen. Einige der restlichen Komponenten bilden dabei das oben erwähnte Haltesystem. Ein solcher Aufbau kann im Servicefall einfach wieder geöffnet und geschlossen werden. In diesem besonderen Aufbau bildet das Filterglas selbst eine wesentliche mechanische Komponente des Strahlergehäuses.

In einer bevorzugten Ausführungsform weist der Strahler Lüftungsöffnungen zur Kühlung auf. Bevorzugt ist in diesem Falle, insbesondere wenn die Lüftung an der Vorderseite des Strahlers liegen soll, dass eine Verklebung auch seitlich oder an der Vorderseite mit den betreffenden Filterglas-Haltevorrichtungen erfolgt, die vorzugsweise Lüftungsöffnungen aufweisen.

In einer weiteren bevorzugten Ausführungsform weist der Strahler Federelemente auf, die insbesondere an dem Filterglas oder der Filterglas-Haltevorrichtung angeordnet sind und den Einbau des Filterglases oder der Filterglas-Haltevorrichtung in ein Haltesystem oder einen sonstigen Aufbau vereinfachen.

Ein besonderer Vorteil der erfindungsgemäßen Infrarotstrahler ist, dass diese besondere Konstruktion auch ganz neue Designaspekte und Erscheinungsformen für Infrarotstrahler eröffnet.

Die Herstellung eines erfindungsgemäßen Infrarotstrahlers erfolgt durch die Schritte:
1. Bereitstellen mindestens eines Filterglases, mindestens einer Filterglas-Haltevorrichtung zusammen mit mindestens einer Infrarotquelle und/oder mindestens einem Haltesystem für eine Infrarotquelle,
2. Verkleben mindestens eines Filterglases mit mindestens einer Filterglas-Haltevorrichtung,
3. Verbinden mindestens einer Filterglas-Haltevorrichtung mit mindestens einer Infrarotquelle und/oder mindestens einem Haltesystem für eine Infrarotquelle.

Beispiele für erfindungsgemäße Infrarotstrahler sind in den Abbildungen dargestellt:
- Figur 1: zeigt einen erfindungsgemäßen Infrarotstrahler;
- Figur 2: zeigt die Anordnung von Filterglas-Haltevorrichtungen in einem erfindungsgemäßen Infrarotstrahler;
- Figuren 3 und 4: zeigen die Verklebung einer Filterglas-Haltevorrichtung an einem Filterglas;
- Figur 5: zeigt einen erfindungsgemäßen Infrarotstrahler;
- Figuren 6 und 7: zeigen Beispiele für den Einbau eines erfindungsgemäßen Infrarotstrahlers.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

Figur 1 zeigt einen rahmenlosen Infrarotstrahler 1 ohne störenden Rand, bei dem Elemente der Filterglas-Haltevorrichtung 2 auf die Rückseite des Filterglases 3 geklebt sind. Diese Elemente stellen gleichzeitig Teile des Gehäuses dar, in dem die Infrarotquelle angeordnet ist.

Die Infrarotquelle kann direkt an den stirnseitigen Filterglas-Haltevorrichtungen angebracht sein oder in den restlichen Gehäuseteilen befestigt sein, die dann als Haltesystem fungieren. Ein solcher Infrarotstrahler stellt sich von außen nur mehr als eine vollkommen, sich beinahe in die Kabinenwand nahtlos einbindende, glatte Glasfläche dar.

In Figur 2 ist ein Filterglas 3 dargestellt auf dessen Oberfläche, vorzugsweise der Rückseite, Filterglas-Haltevorrichtungen 2 aufgeklebt sind.

Mögliche Formen für Klebungen auf der Oberfläche des Filterglases sind in den Figuren 3 und 4 dargestellt. In Figur 3 ist eine mögliche Klebung 4 für Filterglas-Haltevorrichtungen 2 insbesondere auf der Rückseite des Filterglases 3 und in Figur 4 eine Klebung 4 insbesondere auf der Vorderseite dargestellt.

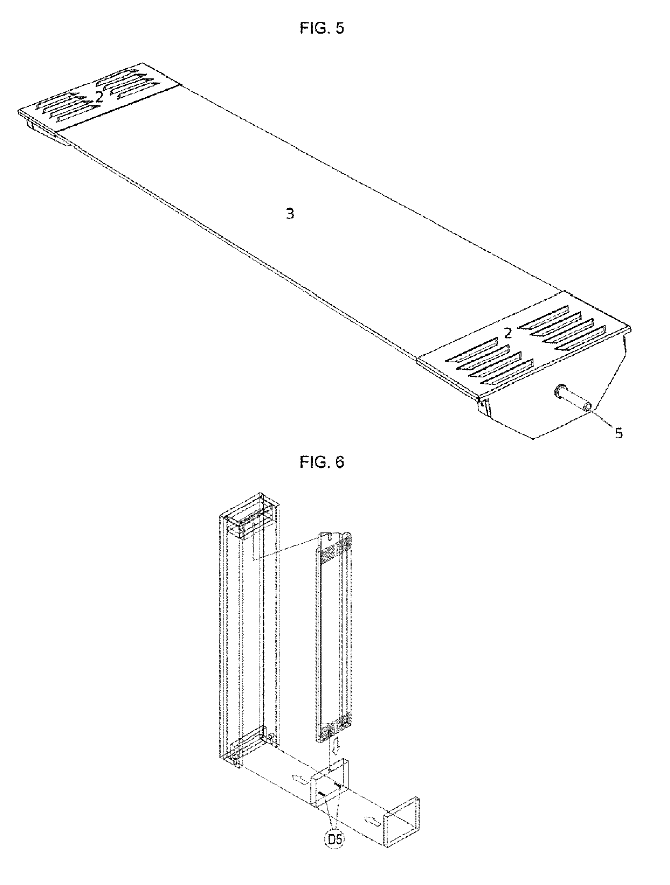

Figur 5 stellt einen Infrarotstrahler dar, bei dem Filterglas-Haltevorrichtungen 2 mit Lüftungsöffnungen zur Ableitung der sich im Inneren des Strahlers bildenden heißen Luft verwendet werden, die in diesem Fall aufgrund der Abwärme der Infrarotquelle zur Verhinderung einer Überhitzung notwendig sind. Auf diese Weise kann eine Verklebung 4 auch seitlich mit Gehäuseteilen oder auf der Vorderseite des Filterglases erfolgen (Figur 4). Dazu von Vorteil ist eine kleine Überlappung des Lüftungsbleches mit dem Glas, welcher durch den Kleber versiegelt wird und hygienisch leicht zu reinigen ist. Dies hat den Vorteil, dass wieder die Längsseiten der Filterglases rahmenlos bleiben und das Filterglas 3 mit den seitlich verklebten Gehäuseteilen mit den Lüftungsöffnungen eine mechanisch stabile Einheit bildet, welche die Strahleraußenmaße auf ein Minimum reduziert.

Es ist auch möglich, die Filterglas-Haltevorrichtungen 2 zusätzlich mit Lagerzapfen 5 für eine drehbare Lagerung auszustatten. Diese Lagerzapfen 5 werden, wie in Figur 6 dargestellt, in entsprechenden Öffnungen eines Strahlerkastens geführt, so dass der Strahler um seine Längsachse drehbar gelagert ist.

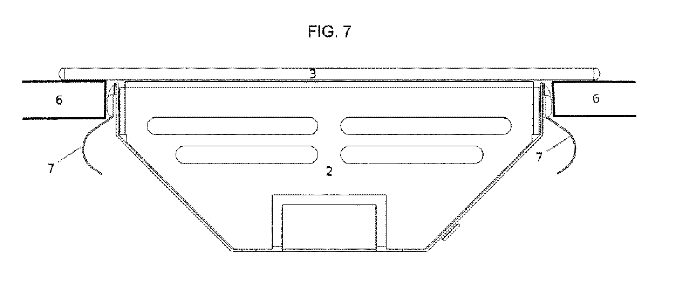

Um die vollkommen glatte Strahlervorderseite bei einem Infrarotstrahler, wie er z.B. in Figur 1 dargestellt ist, nicht zu stören, kann die Befestigung des Strahlers von hinten erfolgen. Da aber nicht immer ein ungehinderter Zugang zur Kabinenrückwand nach erfolgter Kabinenaufstellung gewährt ist, hat sich die Montage der Strahler mittels spezieller Federelemente 7 bewährt. Diese Federelemente 7 befinden sich hinter dem Filterglas 3, auf den Seitenflächen des Strahlergehäuses, vorzugsweise an den Filterglas-Haltevorrichtungen 2 und sind auf die Dicke der Kabinenrückwand 6 abgestimmt. Die Montage erfolgt damit einfach durch Einschieben des Strahlergehäuses in die dafür vorgesehen Ausschnittsöffnung in der Kabinenrückwand 6, bis die Federelemente 7 an der Kabinenrückwand 6 einschnappen und den Strahler sicher halten (Figur 7). Zur Demontage wird der Strahler einfach wieder, gegen die Federkraft, heraus gezogen oder falls zugänglich, von hinten heraus gerückt. Dieser Vorgang ist bei richtiger Wahl der Federelemente 7 sogar mehrmals wiederholbar und sehr servicefreundlich.

Durch einen gewissen Überstand des Filterglases 2 über die Ausschnittsöffnung ist es sogar möglich, die komplette Kabinenwand 6 abzuschließen (siehe Figur 7). Nicht nur, dass bei den bisherigen Strahlern das Strahlergehäuse an sich offen ist, auch die montagebedingten Spalte zwischen Strahlergehäuse und Kabinenwand sind aus hygienischer und thermischer Sicht (Zugluft) nicht optimal.

Der Ordnung halber sei abschließend darauf hingewiesen, dass zum besseren Verständnis des Aufbaus des Infrarotstrahlers dieser bzw. dessen Bestandteile teilweise unmaßstäblich und/oder vergrößert und/oder verkleinert dargestellt wurden.

## Patentansprüche

1. Infrarotstrahler umfassend mindestens ein Filterglas, mindestens eine Infrarotquelle und mindestens eine Filterglas-Haltevorrichtung, **dadurch gekennzeichnet, dass** mindestens eine Filterglas-Haltevorrichtung mit mindestens einem Filterglas verklebt ist.

2. Infrarotstrahler nach Anspruch 1, **dadurch gekennzeichnet, dass** die Filterglas-Haltevorrichtung dazu dient, mindestens ein Filterglas in einer stabilen Position relativ zur Infrarotquelle zu halten und Trägerelemente und/oder Teile eines Gehäuses und/oder eines Reflektors des Infrarotstrahlers sind, und bevorzugt aus Stoffen der Gruppe Metall, Holz, Pappe, Glas und Kunststoff bestehen.

3. Infrarotstrahler nach einem der vorangehenden Ansprüche, **dadurch gekennzeichnet, dass** mindestens eine Infrarotquelle ein Vollspektrumstrahler und/oder Glühdrahtstrahler mit wesentlichen spektralen Anteilen im IR-A und/oder IR-B Bereich ist, und dass das Filterglas zur spektralen Filterung, insbesondere des sichtbaren und/oder des IR-A Anteils, des vom Infrarotemitter emittierten Spektrums dient.

4. Infrarotstrahler nach einem der vorangehenden Ansprüchen, **dadurch gekennzeichnet, dass** der Infrarotstrahler auf einer Seite nur mit dem Filterglas abgeschlossen ist und das Filterglas vorzugsweise keine Öffnungen aufweist und keine Gehäuseteile mit der des Infrarotemitters abgewandten Seite des Glases in Verbindung stehen.

5. Infrarotstrahler nach einem der vorangehenden Ansprüchen, **dadurch gekennzeichnet, dass** das Filterglas rahmenlos mit dem Gehäuse verbunden ist, und dass das Filterglas bevorzugt einen Überstand über das Strahlergehäuse aufweist, so dass nach der Montage des Strahlers der Glasüberstand bündig an der Montagewand aufliegt.

6. Infrarotstrahler nach einem der vorangehenden Ansprüchen, **dadurch gekennzeichnet, dass** die Verklebung auf der der Infrarotquelle zugewandten Seite, deren abgewandter Seite des Filterglases oder am Rand des Filterglases erfolgt, und der Kleber im abgebundenen Zustand eine Flexibilität aufweist.

7. Infrarotstrahler nach einem der vorangehenden Ansprüche, **dadurch gekennzeichnet, dass** mindestens ein Filterglas einen mechanisch tragenden Teil der Gehäusekonstruktion bildet.

8. Infrarotstrahler nach einem der vorangehenden Ansprüche, **dadurch gekennzeichnet, dass** der Infrarotstrahler Federelemente aufweist, mittels derer er, z.B. in einer Montagewand, gehaltert werden kann.

9. Infrarotstrahler nach einem der vorangehenden Ansprüche, **dadurch gekennzeichnet, dass** der Strahler Elemente aufweist, mittels derer er drehbar in einem Strahlerkasten gelagert werden kann.

10. Verfahren zur Herstellung eines Infrarotstrahlers nach einem der vorangehenden Ansprüche **gekennzeichnet durch** die Schritte:
a) Bereitstellen mindestens eines Filterglases, mindestens einer Filterglas-Haltevorrichtung zusammen mit mindestens einer Infrarotquelle und/oder mindestens einem Haltesystem für eine Infrarotquelle,
b) Verkleben mindestens eines Filterglases mit mindestens einer Filterglas-Haltevorrichtung,
c) Verbinden mindestens einer Filterglas-Haltevorrichtung mit mindestens einer Infrarotquelle und/oder mindestens einem Haltesystem für eine Infrarotquelle.
